(19) Europäisches Patentamt
European Patent Office
Office européen des brevets

(11) **EP 3 872 161 A1**

(12) **EUROPEAN PATENT APPLICATION**
published in accordance with Art. 153(4) EPC

(43) Date of publication:
**01.09.2021 Bulletin 2021/35**

(21) Application number: **19891205.7**

(22) Date of filing: **01.11.2019**

(51) Int Cl.:
***C12M 1/00*** *(2006.01)*    ***C12M 1/36*** *(2006.01)*

(86) International application number:
**PCT/JP2019/043025**

(87) International publication number:
**WO 2020/110600 (04.06.2020 Gazette 2020/23)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **29.11.2018 JP 2018223386**

(71) Applicant: **PHC Holdings Corporation
Tokyo 105-8433 (JP)**

(72) Inventors:
• **SAWAI, Akito
Ehime, 791-0395 (JP)**
• **ONDA, Megumi
Ehime, 791-0395 (JP)**

(74) Representative: **Grünecker Patent- und
Rechtsanwälte
PartG mbB
Leopoldstraße 4
80802 München (DE)**

(54) **CULTURE DEVICE**

(57) A culture device comprising: an adjusting gas supplier which supplies to a culture space an adjusting gas for adjusting the concentration of a definite gas component in the culture space; an adjuster which adjusts the moisture in the culture space; and a controller which controls the adjuster, wherein the controller controls the adjuster depending on the supply amount of the adjusting gas or a parameter correlated to the supply amount.

FIG. 3

## Description

Technical Field

The present invention relates to a culture apparatus.

Background Art

In a culture apparatus (incubator) for incubating a culture such as a cell or a microorganism, it is necessary to control various conditions in a culture space such that the conditions are suitable for incubating the culture (see, for example, Patent Literature (hereinafter, referred to as "PTL") 1). Specifically, the conditions such as the temperature, the humidity, the $O_2$ gas concentration, the $CO_2$ gas concentration, and the like in the culture space are controlled.

The gas concentration condition in the culture space is controlled by supplying a gas for adjusting the gas concentration (hereinafter, referred to as "adjustment gas") to the culture space. Air around the culture apparatus is taken in the culture space. Thus, for example, when the set value of $O_2$ concentration is higher than the $O_2$ concentration in the air around the culture apparatus, $O_2$ gas is supplied to the culture space at a flow rate according to the set value of $O_2$ concentration, and, when the set value of $O_2$ concentration is lower than the $O_2$ concentration in the air around the culture apparatus, $N_2$ gas is supplied to the culture space at a flow rate according to the set value of $O_2$ concentration. In addition, $CO_2$ gas is supplied to the culture space at a flow rate according to the set value of $CO_2$ concentration.

Citation List

Patent Literature

PTL 1
Japanese Patent Application Laid-Open No. 2017-201886

Summary of Invention

Technical Problem

However, the humidity of a common adjustment gas is lower than a humidity suitable for culturing. Thus, when the adjustment gas is supplied to the culture apparatus as described above, the humidity in the culture space may fall outside a range suitable for culturing.

The present invention has been devised to solve such a problem, and aims to provide a culture apparatus capable of maintain the humidity in a culture space within an appropriate range.

Solution to Problem

To solve the aforementioned conventional problem, a culture apparatus according to the present invention includes: an adjustment gas supply device that supplies a culture space with an adjustment gas for adjusting a concentration of a predetermined gas component in the culture space; an adjustment device that adjusts a humidity in the culture space; and a controller that controls the adjustment device, in which the controller controls the adjustment device according to a supply amount of the adjustment gas or a parameter correlating with the supply amount.

Advantageous Effects of Invention

According to the present invention, it is possible to maintain the humidity in the culture space within an appropriate range.

Brief Description of Drawings

FIG. 1 illustrates a schematic longitudinal section of a culture apparatus of an embodiment of the present invention as seen from the right side;
FIG. 2 schematically illustrates the back surface of the culture apparatus of an embodiment of the present invention, with a cover being removed;
FIG. 3 is a schematic functional block diagram illustrating a principal part of a control configuration of the culture apparatus of an embodiment of the present invention; and
FIG. 4 is a flowchart illustrating an exemplary control flow of the culture apparatus according to an embodiment of the present invention.

Description of Embodiments

Hereinafter, a culture apparatus according to an embodiment of the present invention will be described with reference to the accompanying drawings. The following embodiments are merely illustrative, and various modifications and/or applications of techniques which are not specified in the following embodiments are not excluded. In addition, the configurations of the embodiments can be variously modified and implemented without departing from the spirit thereof. Further, the configurations of the embodiments can be selected as necessary, or can be appropriately combined.

In the following description, the side of the culture apparatus which the user faces during usage of the culture apparatus (the side with below-described outer door 3a and inner door 3b) is referred to as "front" and the side opposite to the front is referred to as "rear." In addition, the left and right are defined with reference to the case of viewing from the front to the rear.

Note that, in all the figures for explaining the embodiments, the same elements are denoted by the same reference numerals in principle, and the description thereof may be omitted.

[1. Configuration]

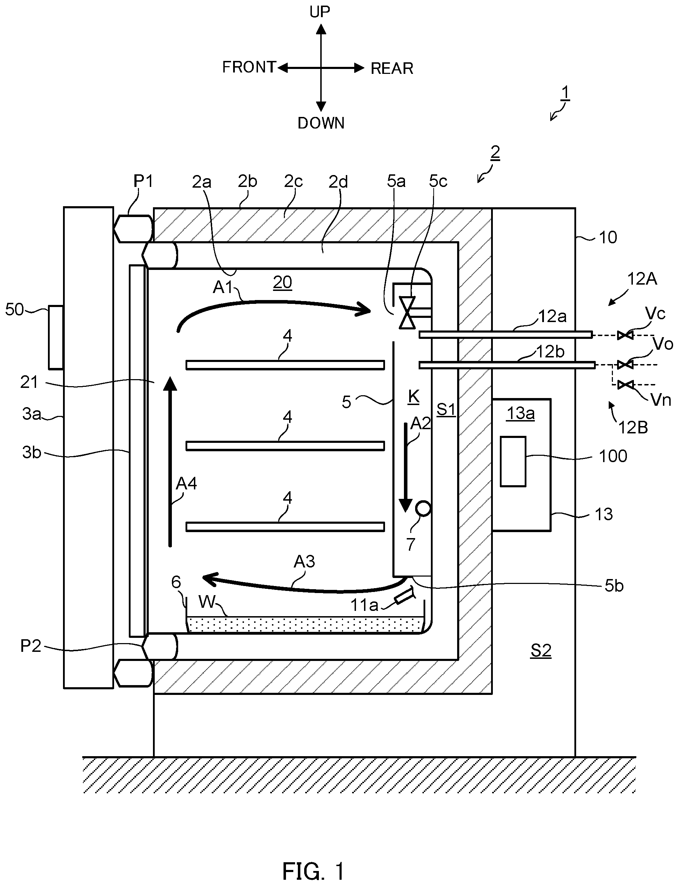

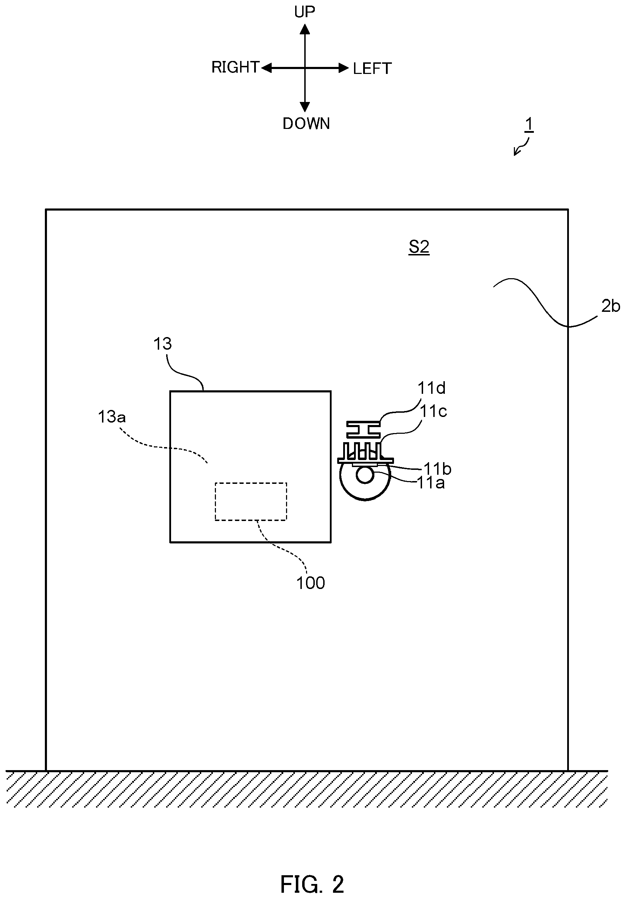

Culture apparatus 1 of an embodiment of the present embodiment will be described with reference to FIGS. 1 and 2. FIG. 1 illustrates a schematic longitudinal section of the culture apparatus of an embodiment of the present invention as seen from the right side. FIG. 2 schematically illustrates the back surface of the culture apparatus of an embodiment of the present embodiment, with a cover being removed.

Culture apparatus 1 illustrated in FIGS. 1 and 2 is an apparatus for growing a culture such as a cell or a microorganism. Culture apparatus 1 is configured to include substantially box-shaped heat insulation box 2 having culture space 20 formed inside and opening 21 formed in the front surface, outer door 3a and inner door 3b for opening and closing opening 21. Culture space 20 is vertically compartmentalized by a plurality of (here, three) shelves 4. Packing P1 is disposed on the outer edge of outer door 3a.

As will be described below, in order to achieve a suitable environment for incubating the culture, culture space 20 is controlled such that the temperature, humidity, $O_2$ (oxygen) concentration, and $CO_2$ (carbon dioxide) concentration are maintained within respective suitable ranges.

Heat insulation box 2 includes substantially box-shaped inner box 2a having culture space 20 formed inside, and substantially box-shaped outer box 2b that covers the outside of inner box 2a.

Outer box 2b is provided, on its inner surface side, with heat insulation material 2c. Space S1 is formed between the inner surface of heat insulation material 2c of outer box 2b and the outer surface of inner box 2a in such a manner as to cover the upper, lower, left, right, and rear sides of inner box 2a. This space S1 is filled with air; the air layer (so-called air jacket) 2d is formed in space S1. Space S1 has an opening in the front, and this opening is sealed with packing P2.

In culture space 20, vertically extending duct 5 is disposed on the rear surface of inner box 2a. Gas passage K is formed inside duct 5. Circulation blower 5c is disposed in gas passage K. By operating circulation blower 5c, air in culture space 20 is sucked through suction port 5a formed in an upper portion of duct 5, and this air is blown out to culture space 20 through blow-out port 5b formed in a lower portion of duct 5. Thus, forced circulation of the air as indicated by arrows A1, A2, A3, and A4 takes place.

In addition, supply ports at the leading ends of respective gas supply pipes 12a and 12b for supplying culture space 20 with an adjustment gas for adjusting an $O_2$ gas concentration and a $CO_2$ gas concentration in

culture space 20 are disposed within duct 5.

Gas supply pipe 12a, a supply line for $CO_2$ gas (adjustment gas) that is connected to gas supply pipe 12a, and $CO_2$ control valve Vc provided midway along this supply line constitute gas supply device 12A for supplying $CO_2$ gas to culture space 20.

Gas supply pipe 12b is a component of gas supply device 12B for supplying $O_2$ gas or $N_2$ gas to culture space 20. Specifically, gas supply pipe 12b, respective supply lines for $O_2$ gas (adjustment gas) and $N_2$ gas (adjustment gas) that are connected to gas supply pipe 12b, and $O_2$ control valve Vo and $N_2$ control valve Vn provided midway along these supply lines constitute gas supply device 12B.

Further, in the present embodiment, UV lamp 7, a temperature sensor for detecting the temperature, an $O_2$ gas sensor for detecting the $O_2$ gas concentration, and a $CO_2$ gas sensor for detecting the $CO_2$ gas concentration are disposed within duct 5 (the temperature sensor, $O_2$ gas sensor, and $CO_2$ gas sensor are not illustrated). Ultraviolet lamp 7 sterilizes water W in humidification tray 6 described later. Note that, the air in duct 5 may be drawn into a space on the rear surface side of the duct, and the temperature sensor, $O_2$ gas sensor, and $CO_2$ gas sensor disposed in the space may perform each detection.

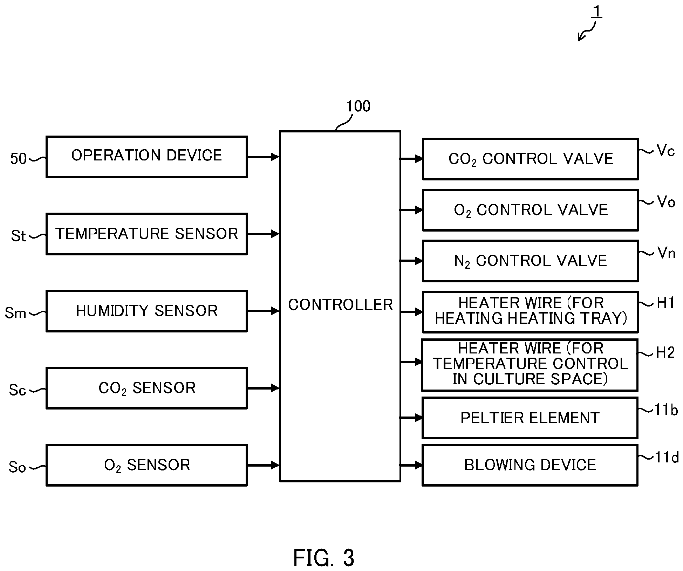

Humidification tray 6 for storing humidification water W is disposed between the lower portion of duct 5 and the bottom plate of inner box 2a. Humidification tray 6 is heated by linear heater (hereinafter referred to as "heater wire") H1 (see FIG. 3) disposed on the bottom plate of inner box 2a. Heated by this heater HI, water W is evaporated to humidify culture space 20. That is, humidification tray 6 and heater wire H1 constitute the "adjustment device that adjusts a humidity in a culture space" of the present invention.

Further, heater wire H2 (see FIG. 3) for controlling the temperature in culture space 20 is disposed on the bottom plate of inner box 2a. In the present embodiment, this heater wire H2 and above-described heater wire H1 that heats humidification tray 6 are provided separately, and the outputs (temperatures) are individually controlled.

Note that, heater wire H1 for heating humidification tray 6 and heater wire H2 for controlling the temperature in culture space 20 may be integrally provided.

In addition, culture apparatus 1 receives instructions to start and stop culture apparatus 1 and/or inputs of various set values for culture space 20 from operation device 50 disposed on outer door 3a. The various set values for culture space 20 include a set temperature, a set concentration of $O_2$ gas (hereinafter referred to as "$O_2$ concentration detection value"), a set concentration of $CO_2$ gas (hereinafter referred to as "$CO_2$ concentration detection value"), and the like. Below-described controller 100 controls the temperature, humidity, $O_2$ concentration, $CO_2$ concentration, and the like in culture space 20 to be the above-mentioned set values.

The back and bottom surfaces of outer box 2b of heat insulation box 2 are covered with cover 10. The space between the back surface of outer box 2b and cover 10 forms mechanical room S2 for disposing various equipment therein. Electrical box 13 is disposed in mechanical room S2. Controller 100 and other electrical components (not illustrated) are housed in inner space 13a of electrical box 13.

Further, as illustrated in FIG. 2, dew condensation member 11a is disposed on the back surface of heat insulation box 2. This dew condensation member 11a is inserted into culture space 20 from mechanical room S2. It is preferable that dew condensation member 11a have higher conductivity, and the dew condensation member is, for example, a round bar with a predetermined length that is made of aluminum, silver or the like. Peltier element 1 1b is disposed on an end of dew condensation member 11a within mechanical room S2 such that the heat-absorbing surface of the Peltier element faces the end. The dew condensation member is cooled by the heat-absorbing surface. Accordingly, condensation water is generated on the surface of dew condensation member 11a in culture space 20, and consequently, the humidity in culture space 20 can be controlled within a predetermined range. Note that, the condensation water generated on the surface of dew condensation member 11a drips from the tip of dew condensation member 11a into humidification tray 6.

Further, comb-shaped heat sink 11c disposed on the heating surface of Peltier element 11b, and blowing device 11d for supplying a cooling wind toward this heat sink 11c are disposed in mechanical room S2. The cooling wind (air) blown from blowing device 11d cools the heating surface of Peltier element 11b via heat sink 11c.

[2. Control Configuration]

Hereinafter, a principal part of a control configuration of culture apparatus 1 of an embodiment of the present invention will be described with reference to FIG. 3.

FIG. 3 is a schematic functional block diagram illustrating the principal part of the control configuration of culture apparatus 1 of an embodiment of the present invention.

As illustrated in FIG. 3, controller 100 receives control signals from operation device 50, temperature sensor St, humidity sensor Sm, $CO_2$ sensor Sc, and $O_2$ sensor So. Further, controller 100 outputs control commands to control valves Vc, Vo, and Vn, heater wires H1 and H2, and Peltier element 11b, and blowing device 11d.

Here, operation device 50 receives inputs of various settings for culture space 20. Specifically, various settings such as set temperature T, set $CO_2$ concentration x [%], and set $O_2$ concentration y [%] in culture space 20 are inputted to operation device 50. Operation device 50 inputs these settings to controller 100 as control signals.

In addition, temperature sensor St detects the temperature in culture space 20 and outputs the detected temperature to controller 100 as a control signal. Humidity sensor Sm detects the humidity in culture space 20 and outputs the detected humidity to controller 100 as a control signal. $O_2$ sensor So detects the $O_2$ concentration in culture space 20 and outputs the detected $O_2$ concentration to controller 100 as a control signal. $CO_2$ sensor Sc detects the $CO_2$ concentration in culture space 20 and outputs the detected $CO_2$ concentration to controller 100 as a control signal.

Controller 100 controls the valve opening degree or duty ratio (on-off ratio) of $CO_2$ control valve Vc based on the $CO_2$ concentration detected by $CO_2$ sensor Sc, to control flow rate Fc of $CO_2$ gas per unit time. Specifically, when the $CO_2$ concentration detection value is lower than set $CO_2$ concentration x by a predetermined value or a value greater than the predetermined value, the valve opening degree or duty ratio of $CO_2$ control valve Vc is controlled such that flow rate Fc increases. Conversely, when the $CO_2$ concentration detection value is higher than set $CO_2$ concentration x by a predetermined value or a value greater than the predetermined value, the valve opening degree or duty ratio of $CO_2$ control valve Vc is controlled such that flow rate Fc decreases.

When set $O_2$ concentration y is higher than the $O_2$ concentration (usually about 20%) in the air around culture apparatus 1, that is, the air to be taken into culture space 20, controller 100 outputs a control command to $O_2$ control valve Vo to cause $O_2$ control valve Vo to be opened for supplying $O_2$ gas to culture space 20. At this time, controller 100 controls the valve opening degree or duty ratio of $O_2$ control valve Vo based on the $O_2$ concentration detected by $O_2$ sensor So, to control flow rate Fo of $O_2$ gas per unit time. Specifically, when the $O_2$ concentration detection value is lower than set $O_2$ concentration y by a predetermined value or a value greater than the predetermined value, the valve opening degree or duty ratio of $O_2$ control valve Vo is controlled such that flow rate Fo increases, and, when the $O_2$ concentration detection value is higher than set $O_2$ concentration y by a predetermined value or a value greater than the predetermined value, the valve opening degree or duty ratio of $O_2$ control valve Vo is controlled such that flow rate Fo decreases.

On the other hand, when set $O_2$ concentration y is lower than that in the air around culture apparatus 1, controller 100 outputs a control command to $N_2$ control valve Vn to cause $N_2$ control valve Vn to be opened for supplying $N_2$ gas to culture space 20. At this time, controller 100 controls the valve opening degree or duty ratio of $N_2$ control valve Vn based on the $O_2$ concentration detection value, to control flow rate Fn of $N_2$ gas per unit time. Specifically, when the $O_2$ concentration detection value is lower than set $O_2$ concentration y by a predetermined value or a value greater than the predetermined value, the valve opening degree or duty ratio of $N_2$ control valve Vn is controlled such that flow rate Fn decreases, and, when the $O_2$ concentration detection value is higher than set $O_2$ concentration y by a predetermined value or a value greater than the predetermined value, the valve opening degree or duty ratio of $N_2$ control valve Vn is controlled such that flow rate Fn increases.

Note that, $O_2$ control valve Vo and $N_2$ control valve Vn may be configured by a single control valve. For example, a control valve that causes a port for supplying $O_2$ and a port for supplying $N_2$ to selectively communicate with a port for gas supply to gas supply pipe 12b may be used as the single control valve.

Further, controller 100 controls the output of heater wire H2 based on the temperature in culture space 20 detected by temperature sensor St (hereinafter referred to as "temperature detection value"). Specifically, the energization rate of heater wire H2 is controlled such that the temperature of heater wire H2 increases when the temperature detection value is lower than set temperature T by a predetermined value or by a value greater than the predetermined value, or such that the temperature of heater wire H2 decreases when the temperature detection value is higher than set temperature T by a predetermined value or by a value greater than the predetermined value.

Further, controller 100 controls the adjustment device for adjusting the humidity such that the humidity in culture space 20 does not fall outside expected range R (hereinafter, referred to as "appropriate range R") of humidity where the humidity is higher than that in the air around culture apparatus 1 and is suitable for incubating a culture. That is, at least one of the outputs of heater wire HI, Peltier element 11b and blowing device 11d is controlled.

Here, a common $CO_2$ gas supplied to culture space 20 as the adjustment gas in order to change the $CO_2$ concentration in culture space 20 to the set concentration has a humidity lower than the humidity in appropriate range R. Accordingly, the humidity in culture space 20 may fall below appropriate range R. Similarly, a common $O_2$ gas and a common $N_2$ gas supplied to culture space 20 as the adjustment gas in order to change the $O_2$ concentration in culture space 20 to the set concentration have humidities lower than those in appropriate range R, respectively. Therefore, the humidity in culture space 20 may fall below appropriate range R.

Accordingly, when the adjustment gas is supplied to culture space 20, controller 100 performs a humidity maintaining control for compensating a decrease in humidity caused by the supply of the adjustment gas. In the humidity maintaining control, at least one of the following control 1, control 2, and control 3 is performed according to the supply amount of the adjustment gas or a parameter correlating with the supply amount (hereinafter referred to as "correlation parameter"). Examples of the correlation parameter include the sum (= x + z) of set $CO_2$ concentration x and set $NO_2$ concentration z (=

100 - set $CO_2$ concentration x - set $O_2$ concentration y).

(1) Control 1

In control 1, controller 100 increases the output (heating amount) of heater wire H1 to an output greater than in a case where the adjustment gas is not supplied to culture space 20. Specifically, the energization rate of heater wire H1 is increased stepwise or continuously with increasing supply amount or correlation parameter of the adjustment gas. Thus, the evaporation amount of water W in humidification tray 6 due to heating by heater wire H1 increases, the decrease in humidity due to the supply of the adjustment gas is compensated, and the humidity in culture space 20 is maintained within the appropriate range.

(2) Control 2

In control 2, controller 100 decreases the output of Peltier element 11b to an output less than in a case where the adjustment gas is not supplied to culture space 20. Specifically, an applied voltage to Peltier element 11b is lowered stepwise or continuously with increasing supply amount or correlation parameter of the adjustment gas. Thus, the output (cooling amount) of Peltier element 1 1b is lowered, the temperature of dew condensation member 11a is increased accordingly, and the humidity in culture space 20 is relatively increased as compared with the case where the adjustment gas is not supplied to culture space 20. Consequently, the decrease in humidity due to the supply of the adjustment gas is compensated, and the humidity in culture space 20 is maintained within the appropriate range.

(3) Control 3

In control 3, controller 100 decreases the output of blowing device 11d to an output less than in a case where the adjustment gas is not supplied to culture space 20. Specifically, a fan rotation speed of blowing device 11d is lowered stepwise or continuously with increasing supply amount or correlation parameter of the adjustment gas. Accordingly, the amount of air blown from blowing device 11d to the heating surface of Peltier element 11b is reduced, and the cooling amount for the heating surface is reduced. Consequently, the heat conversion efficiency of Peltier element 11b is lowered, the temperature of the heat-absorbing surface of Peltier element 11b and, thus, the temperature of dew condensation member 11a are increased, and the humidity in culture space 20 is relatively increased as compared with the case where the adjustment gas is not supplied to culture space 20. Consequently, the decrease in humidity due to the supply of the adjustment gas is compensated, and the humidity in culture space 20 is maintained within the appropriate range.

[3. Control Flow]

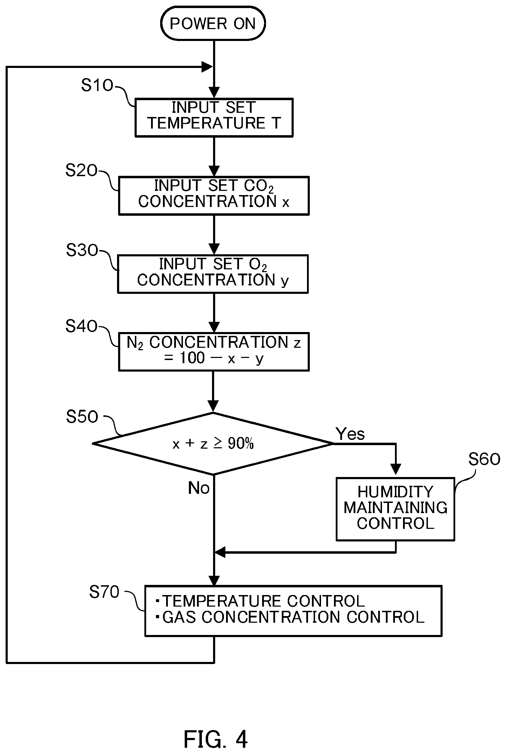

A description will be given of a control flow of the culture apparatus of an embodiment of the present invention with reference to FIG. 4. FIG. 4 is a flowchart illustrating an exemplary control flow of the culture apparatus according to an embodiment of the present invention. Note that, this control flow assumes that set $O_2$ concentration y is lower than the $O_2$ concentration in the air around culture apparatus 1, and $N_2$ gas instead of $O_2$ gas is supplied to culture space 20 as the adjustment gas.

This control flow is started when culture apparatus 1 is started (powered on) by the operation of operation device 50, and is repeated until culture apparatus 1 is stopped (powered off).

When culture apparatus 1 is started, controller 100 receives the input of set temperature T at step S10, the input of set $CO_2$ concentration x at step S20, the input of set $O_2$ concentration y at step S30 by operation device 50.

Next, controller 100 obtains $N_2$ concentration z [%] from set $CO_2$ concentration x [%] and set $O_2$ concentration y [%] at step S40 in accordance with following Equation 1:

$$z = 100 - x - y \qquad \dots \text{(Equation 1)}.$$

Then, at step S50, controller 100 determines whether following Equation 2 is satisfied:

$$x + y \geqq 90 \qquad \dots \text{(Equation 2)}.$$

That is, controller 100 obtains the total value of set $CO_2$ concentration x and $N_2$ concentration z as the correlation parameter correlating with the supply amount of the adjustment gas, and determines whether or not the total value is equal to or greater than a predetermined value (90% in the present embodiment). When the total value does not exceed 90%, controller 100 performs a temperature control of changing the temperature in culture space 20 to set temperature T, and a gas concentration control of changing the $CO_2$ concentration and the $O_2$ concentration in culture space 20 to set $CO_2$ concentration x and set $O_2$ concentration y at step S70. Meanwhile, when the total value exceeds 90%, controller 100 performs the above-described humidity maintaining control at step S60, and then proceeds to aforementioned step S70. After performing the control at step S70, controller 100 repeats the processing of step S10 and subsequent steps.

Note that, when $O_2$ gas instead of $N_2$ gas is supplied to culture space 20 as the adjustment gas, it is determined whether or not the total value of set $CO_2$ concentration x and set $O_2$ concentration y is equal to or greater than a predetermined value at step S50.

[4. Effects]

Controller 100 controls, according to the supply amount of the adjustment gas, the adjustment device that adjusts the humidity in culture space 20. In the present embodiment, at least one of $CO_2$ gas, $O_2$ gas, and $N_2$ gas is used as the adjustment gas, and heater wire H2, Peltier element 11b, and blowing device 11d are controlled as the adjustment device according to the parameter correlating with the supply amount of the adjustment gas. Further, when a plurality of kinds of adjustment gases (e.g., $CO_2$ gas and $N_2$ gas) are supplied simultaneously, the sum of the supply amounts of the supplied adjustment gases corresponds to the supply amount of the adjustment gas.

Specifically, when the total value of set $CO_2$ concentration x and $N_2$ concentration z, which is the parameter correlating with the supply amount of the adjustment gas, is equal to or greater than a predetermined value, the supply amount of the adjustment gas of a low humidity is large and the humidity in culture space 20 decreases. Accordingly, at least one of heater wire H2, Peltier element 11b, and blowing device 11d is controlled such that the humidity in culture space 20 becomes higher than that in the case where the adjustment gas is not supplied.

Thus, according to the present invention, it is possible to maintain the humidity in the culture space within an appropriate range even when the adjustment gas is supplied to the culture space.

[5. Modifications]

(1) When the total value of set supply amounts or actual supply amounts of $CO_2$ gas, $O_2$ gas, and $N_2$ gas exceeds a predetermined value, the humidity maintaining control may be performed.

(2) When the total value of valve opening degrees or the total value of duty ratios of $CO_2$ control valve Vc, $O_2$ control valve Vo, and $N_2$ control valve Vn that control the supply amounts of $CO_2$ gas, $O_2$ gas, and $N_2$ gas to culture space 20 exceeds a predetermined value, the humidity maintaining control may be performed.

(3) When the total value of an increase in set $CO_2$ concentration x and the absolute value of an increase or a decrease in set $O_2$ concentration x with respect to the concentrations of respective components of $CO_2$ and $O_2$ in the air around culture apparatus 1 exceeds a predetermined value, the humidity maintaining control may be performed. Note that, the absolute value of the increase or decrease in set $O_2$ concentration x is used for calculation because when set $O_2$ concentration x is higher than that in the ambient air, $O_2$ gas is supplied, and when set $O_2$ concentration x is lower than that in the ambient air, $N_2$

gas is supplied. That is, whether set $O_2$ concentration x is high or low with respect to the $O_2$ concentration in the ambient air, the adjustment gas is supplied, and the humidity in the air in culture apparatus 1 decreases accordingly.

A description will be given of an example on the assumption that concentration x0 of $CO_2$ and concentration y0 of $O_2$ in the air around culture apparatus 1 are 0% and 20%, respectively, and set $CO_2$ concentration x is 30% and set $O_2$ concentration y is 30%. For $CO_2$, difference $\Delta x$ (= x - x0) between concentration x0 and set $CO_2$ concentration x is 30%, and for $O_2$, the absolute value of difference $\Delta y$ between concentration y0 and set $O_2$ concentration y (= |y - y0|) is 10%. Total value T of these values is 40%. When total value T of 40% exceeds a predetermined value, the humidity maintaining control is performed.

In addition, a description will be given of an example on the assumption that concentration x0 of $CO_2$ and concentration y0 of $O_2$ in the air around culture apparatus 1 are 0% and 20%, respectively, and set $CO_2$ concentration x is 30% and set $O_2$ concentration y is 10%. For $CO_2$, difference $\Delta x$ (= x - x0) between concentration x0 and set $CO_2$ concentration x is 30%, and for $O_2$, the absolute value of difference $\Delta y$ between concentration y0 and set $O_2$ concentration y (= |y - y0|) is 10%. Total value T of these values is 40%. When total value T of 40% exceeds a predetermined value, the humidity maintaining control is performed.

In the above embodiment, when the sum (= x + z) of set $CO_2$ concentration x and set $NO_2$ concentration z exceeds a predetermined value, the humidity maintaining control is performed (see steps S50 and S60 in FIG. 4). A plurality of predetermined values may be set, and the level of the humidity maintaining control may be raised stepwise each time the sum (= x + z) exceeds a predetermined value. Alternatively, the level of humidity maintaining control may be raised continuously (linearly) as the sum increases.

Raising the level of the humidity maintaining control stepwise or continuously means at least one of increasing the output of heater wire HI, decreasing the output of Peltier element 11b, and decreasing the output of blowing device 11d stepwise or continuously. Alternatively, when the level of the humidity maintaining control is raised stepwise, increasing the output of heater wire HI, decreasing the output of Peltier element 11b, and decreasing the output of blowing device 11d may be added sequentially. Specifically, it is conceivable that, when the sum (= x + z) exceeds a first predetermined value, the output of heater wire H1 is increased, and then, when the sum exceeds a second predetermined value greater than the first predetermined value, the output of Peltier element 11b is decreased in addition to increasing the output of heater wire H1.

Note that, as one exemplary embodiment, the above embodiment has been described which has the configuration including humidity sensor Sm, but humidity sensor Sm is not necessarily required.

By performing the humidity control in the case where the adjustment gas is supplied as described in the above embodiment, the humidity control can be performed at an earlier stage than a humidity control based on a detected value of humidity detected using a humidity sensor after the humidity changes. That is, it is possible to deal with a change in the humidity value while expecting the change in advance.

The disclosure of Japanese Patent Application No. 2018-223386, filed on November 29, 2018, including the specification, claims, drawings, and abstract is incorporated herein by reference in its entirety.

Industrial Applicability

The present invention is suitably utilized as a culture apparatus.

Reference Signs List

1 Culture apparatus
2 Heat insulation box
2a Inner box
2b Outer box
2c Heat insulation material
2d Air layer
3 a Outer door
3b Inner door
4 Shelf
5 Duct
5a Suction port
5b Blow-out port
5c Circulation blower
6 Humidification tray
7 Ultraviolet lamp
10 Cover
11a Dew condensation member
11b Peltier element
11c Heat sink
11d Blowing device
12A, 12B Gas supply device
13 Electrical box
13a Inner space
20 Culture space
21 Opening
50 Operation device
100 Controller
K Gas passage
P I, P2 Packing
S1 Space (first space)
S2 Mechanical room
W Water
Vc $CO_2$ control valve
Vn $N_2$ control valve

Vo   $O_2$ control valve

according to the supply amount or the parameter correlating with the supply amount.

**Claims**

1. A culture apparatus, comprising:

    an adjustment gas supply device that supplies a culture space with an adjustment gas for adjusting a concentration of a predetermined gas component in the culture space;
    an adjustment device that adjusts a humidity in the culture space; and
    a controller that controls the adjustment device, wherein
    the controller controls the adjustment device according to a supply amount of the adjustment gas or a parameter correlating with the supply amount.

2. The culture apparatus according to claim 1, wherein the adjustment device is configured to comprise:

    a humidification tray that is disposed in the culture space and stores a liquid, and
    a heater for heating the humidification tray to evaporate the liquid, and

    the controller controls an output of the heater according to the supply amount or the parameter correlating with the supply amount.

3. The culture apparatus according to claim 1 or 2, wherein
    the adjustment device comprises a dew condensation mechanism configured to condense moisture in the culture space, and
    the controller controls the dew condensation mechanism according to the supply amount or the parameter correlating with the supply amount.

4. The culture apparatus according to claim 3, wherein the dew condensation mechanism comprises:

    a dew condensation member at least partially disposed in the culture space, and
    a Peltier element configured to cool the dew condensation member, and

    the controller controls an output of the Peltier element according to the supply amount or the parameter correlating with the supply amount.

5. The culture apparatus according to claim 4, wherein the dew condensation mechanism further comprises a blowing device configured to blow gas to a heating surface of the Peltier element, and
    the controller controls an output of the blowing device

UP

FRONT ← → REAR

DOWN

1

2

P1  2a  2b  2c  2d  5a  5c

10

12A

50

A1  20

12a  Vc

12b  Vo

21

Vn

3a

5  K  S1

12B

3b

4  A2

13a

100

A4

4

7

13

A3

6

5b

W

11a

S2

P2

FIG. 1

UP

RIGHT ← → LEFT

DOWN

1

S2

2b

13

11d
11c

13a

11b
11a

100

FIG. 2

$\underset{\sim}{1}$

100

| 50 | OPERATION DEVICE |
| St | TEMPERATURE SENSOR |
| Sm | HUMIDITY SENSOR |
| Sc | $CO_2$ SENSOR |
| So | $O_2$ SENSOR |

CONTROLLER

| $CO_2$ CONTROL VALVE | Vc |
| $O_2$ CONTROL VALVE | Vo |
| $N_2$ CONTROL VALVE | Vn |
| HEATER WIRE (FOR HEATING HEATING TRAY) | H1 |
| HEATER WIRE (FOR TEMPERATURE CONTROL IN CULTURE SPACE) | H2 |
| PELTIER ELEMENT | 11b |
| BLOWING DEVICE | 11d |

FIG. 3

```
                        ┌──────────────┐
                        │   POWER ON   │
                        └──────────────┘
                                │
                                ▼
        S10 ──┐       ┌────────────────────┐
              └───────│    INPUT SET       │
                      │   TEMPERATURE T    │
                      └────────────────────┘
                                │
                                ▼
        S20 ──┐       ┌────────────────────┐
              └───────│  INPUT SET CO₂     │
                      │  CONCENTRATION x   │
                      └────────────────────┘
                                │
                                ▼
        S30 ──┐       ┌────────────────────┐
              └───────│  INPUT SET O₂      │
                      │  CONCENTRATION y   │
                      └────────────────────┘
                                │
                                ▼
        S40 ──┐       ┌────────────────────┐
              └───────│ N₂ CONCENTRATION z │
                      │   = 100 − x − y    │
                      └────────────────────┘
                                │
                                ▼
        S50 ──┐         ◇─────────────◇
              └────────◇  x + z ≥ 90%  ◇────── Yes ──┐
                        ◇─────────────◇              │
                                │                    ▼
                               No           ┌──────────────────┐  ┌── S60
                                │            │    HUMIDITY      │
                                │            │   MAINTAINING    │
                                │            │    CONTROL       │
                                │            └──────────────────┘
                                │                    │
                                ◄────────────────────┘
                                │
                                ▼
        S70 ──┐       ┌─────────────────────────────────┐
              └───────│ ·TEMPERATURE CONTROL            │
                      │ ·GAS CONCENTRATION CONTROL      │
                      └─────────────────────────────────┘
```

FIG. 4

**EP 3 872 161 A1**

## INTERNATIONAL SEARCH REPORT

| International application No. |
|---|
| PCT/JP2019/043025 |

### A. CLASSIFICATION OF SUBJECT MATTER
Int.Cl. C12M1/00(2006.01)i, C12M1/36(2006.01)i
FI: C12M1/00C, C12M1/36

According to International Patent Classification (IPC) or to both national classification and IPC

### B. FIELDS SEARCHED

Minimum documentation searched (classification system followed by classification symbols)
Int.Cl. C12M1/00-3/10

Documentation searched other than minimum documentation to the extent that such documents are included in the fields searched
Published examined utility model applications of Japan 1922-1996
Published unexamined utility model applications of Japan 1971-2020
Registered utility model specifications of Japan 1996-2020
Published registered utility model applications of Japan 1994-2020

Electronic data base consulted during the international search (name of data base and, where practicable, search terms used)
JSTPlus/JMEDPlus/JST7580(JDreamIII),
CAplus/MEDLINE/EMBASE/BIOSIS/WPIDS(STN)

### C. DOCUMENTS CONSIDERED TO BE RELEVANT

| Category* | Citation of document, with indication, where appropriate, of the relevant passages | Relevant to claim No. |
|---|---|---|
| X | JP 2003-029164 A (TOKKEN KK) 29 January 2003, claims, paragraphs [0022], [0032]-[0051], [0058], fig. 1, | 1 |
| Y | claims, paragraphs [0022], [0023], [0032]-[0051], [0058], fig. 1, | 1-2 |
| A | entire text | 3-5 |
| Y | JP 07-031463 A (SANYO ELECTRIC CO., LTD.) 03 February 1995, paragraph [0008], fig. 1, 2 | 1-5 |
| Y | JP 2016-059363 A (PANASONIC HEALTHCARE HOLDINGS CO., LTD.) 25 April 2016, claims, paragraphs [0028]-[0034], [0053]-[0057], [0090], fig. 2-9 | 1-5 |
| Y | JP 2017-175944 A (PANASONIC HEALTHCARE HOLDINGS CO., LTD.) 05 October 2017, paragraph [0016] | 1-2 |
| A | entire text | 3-5 |

☒ Further documents are listed in the continuation of Box C. ☒ See patent family annex.

| * Special categories of cited documents: | "T" later document published after the international filing date or priority date and not in conflict with the application but cited to understand the principle or theory underlying the invention |
|---|---|
| "A" document defining the general state of the art which is not considered to be of particular relevance | |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
|---|---|
| 15.01.2020 | 28.01.2020 |

| Name and mailing address of the ISA/ | Authorized officer |
|---|---|
| Japan Patent Office 3-4-3, Kasumigaseki, Chiyoda-ku, Tokyo 100-8915, Japan | Telephone No. |

Form PCT/ISA/210 (second sheet) (January 2015)

**INTERNATIONAL SEARCH REPORT**

| International application No. |
| --- |
| PCT/JP2019/043025 |

C (Continuation). DOCUMENTS CONSIDERED TO BE RELEVANT

| Category* | Citation of document, with indication, where appropriate, of the relevant passages | Relevant to claim No. |
| --- | --- | --- |
| Y | JP 2005-118021 A (SANYO ELECTRIC CO., LTD.) 12 May 2005, claims, paragraphs [0004], [0005], [0016], examples, fig. 2 | 1-5 |
| A | JP 2017-201886 A (AIREX CO., LTD.) 16 November 2017, entire text | 1-5 |
| A | JP 2005-198565 A (NIKON CORPORATION) 28 July 2005, entire text | 1-5 |
| A | JP 11-089561 A (KUREA KK) 06 April 1999, entire text | 1-5 |

Form PCT/ISA/210 (second sheet) (January 2015)

**INTERNATIONAL SEARCH REPORT**
Information on patent family members

International application No.

PCT/JP2019/043025

| | | |
|---|---|---|
| JP 2003-029164 A | 29 January 2003 | (Family: none) |
| JP 07-031463 A | 03 February 1995 | US 5519188 A<br>column 5, lines 15-29,<br>fig. 1, 2 |
| JP 2016-059363 A | 25 April 2016 | (Family: none) |
| JP 2017-175944 A | 05 October 2017 | (Family: none) |
| JP 2005-118021 A | 12 May 2005 | US 2005/0084420 A1<br>claims, paragraphs [0004],<br>[0005], [0007], examples,<br>fig. 2 |
| JP 2017-201886 A | 16 November 2017 | (Family: none) |
| JP 2005-198565 A | 28 July 2005 | (Family: none) |
| JP 11-089561 A | 06 April 1999 | (Family: none) |

Form PCT/ISA/210 (patent family annex) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2017201886 A
- JP 2018223386 A